# EUROPEAN PATENT SPECIFICATION

(11) **EP 0 572 483 B1**
(45) Date of publication and mention of the grant of the patent: **07.12.1994**
(21) Application number: 92905360.1
(22) Date of filing: 17.02.1992
(51) Int. Cl.: A01N 25/34, A01N 43/16, A01N 47/30, C11D 3/48

(54) **CLEANER**
REINIGUNGSMITTEL
PODUIT DE NETTOYAGE

(30) Priority: 18.02.1991 SE 9100480
(43) Date of publication of application: 08.12.1993
(73) Proprietor: MEDSCAND AB, S-20074 Malmö (SE)
(72) Inventor: FORSGREN, Arne, S-230 11 Falsterbo (SE); LUNDBLAD, Arne, S-756 45 Uppsala (SE); LÖNN, Hans, S-222 41 Lund (SE)
(74) Representative: Ström, Tore
(86) International application number: SE9200093
(87) International publication number: WO9214361

(56) References cited:
- EP-A- 0 126 043
- EP-A- 0 221 728
- WO-A-87/01400
- WO-A-89/05093
- DE-A- 3 408 653
- SE-B- 463 314
- CHEMICAL ABSTRACTS, vol. 91, no. 1, 2 July 1979, Columbus, Ohio, US; M. ARONSO et al.: "Prevention of colonization of the urinary tract of mice with Escherichia coli by blocking of bacterial adherence with methyl x-D-mannopyranoside", see p. 36, abstract 322k
- CHEMICAL ABSTRACTS, vol. 112, no. 19, 7 May 1990, Columbus, Ohio, US; Toyota, Seiichi et al.: "Anti-bacterial defense mechanism of the urinary bladder: role of mannose in urine", see p. 578, abstract 176889s
- CHEMICAL ABSTRACTS, vol. 90, no. 15, 9 April 1979, Columbus, Ohio, US; Eshdat, Yuval et al.: "Isolation of a mannose-specific lectin from Escherichia coli and its role in the adherence of the bacteria to epithelial cells", see p. 470, abstract 119508v

## Description

The present invention refers to a new cleansing product which can be used for effective removal of bacteria, especially type-1 fimbriated E. coli, when wiping for example the hands, in connection with personal hygiene, or the like.

A variety of products are known intended for wiping, cleansing etc., which either can be of the dry type, as for example facial tissues of different kinds, or of the wet type, so called wet wipes. The latter are supplied enclosed in a sealed envelope in order to prevent premature evaporation. All these known products are well suited for the intended purpose but fail in efficiency regarding the effective removal of bacteria from the site concerned. This applies especially to E. coli bacteria, when type-1 fimbriated.

The purpose of previously known cleansing products interacting with bacteria, such as wiping cloths, is to generally inactivate or kill the bacteria. Thus, WO 8905093 discloses a wipe which releases agents for disinfection. WO 8701400 discloses a web containing an antimicrobial substance which is substantive to the fibres of the web for the purpose of preventing the diffusion of the antimicrobioal substance.

One disadvantage of such known cleansing products is that they can cause side-effects which in general are rapidly passing but also can be of a more serious nature.

During the last years the bonding of carbohydrates to bacteria has been a well studied phenomenon. The specific binding of type-1 fimbriated E. coli to mannose has among others been studied by Feron et al. ("Carbohydrate binding sites of the mannose-specific fimbrial lectins of enterobacteria", Infection and Immunity, vol 43, 1984, pp. 1088-90 and "Carbohydrate specificity of the surface lectins of E. coli, K. pneumonia, S. typhimurium", Carbohydrate Research, vol 120, 1983, pp. 235-249).

DE 34 08 653 discloses a deodorant body cleansing composition which comprises at least one carbohydrate selected from glucose, mannose and oligomers thereof, as agent for reducing the odour-causing bacterial population on the body in an anionic detergent-containing vehicle.

The main purpose of the present invention is to achieve a new type of cleansing product which makes it possible to effectively remove bacteria, especially type-1 fimbriated E. coli, in connection with the intended use, for example when wiping the hands or in connection with personal hygiene etc.

Another purpose of the invention is the achievement of a cleansing product of the intended kind, which contains an active component interacting with those bacteria which are intended to be removed and which thus can be said to represent the receptor for these bacteria.

Still another purpose of the invention is to achieve a cleansing product of this kind where the active component is represented by a carbohydrate structure.

In connection with research and experimental work it has thus unexpectedly been shown that if a mannose derivate, especially a mannoside, is bound to the matrix of the cleansing product an effective removal of the bacteria in question from the cleaning site is achieved.

It is especially preferred to use p-aminophenyl-α-D-mannopyranoside as the mannose derivate.

The binding to the matrix can take place in an arbitrary way and can consist of a non-specific adhesion to the matrix of the cleansing product. Such adhesion can be achieved by simply dipping the matrix in a solution containing the active component which is sucked up into the material and after drying of the material remains there.

It is, however, especially preferred to more specifically bind the mannopyranoside via its p-amino group to the product matrix. Such a bond is preferably a covalent bond, either directly to the p-amino group of the mannoside or indirectly via any kind of linker, as is well-known in the art.

Another way of binding the active component to the cleansing product according to the invention is to integrate it by copolymerization which in this latter case suitably consists of a composite material comprising for example cellulose and/or regenerated cellulose and a synthetic copolymer in which the carbohydrate structure is included. Such a copolymer can be constructed with a mannose derivate and an amide as starting materials. Especially, the copolymer can have the formula: 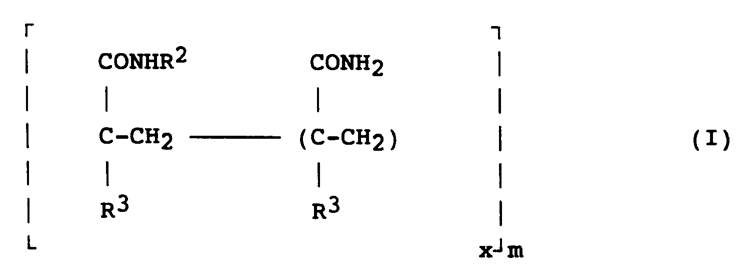
in which R² has the formula: 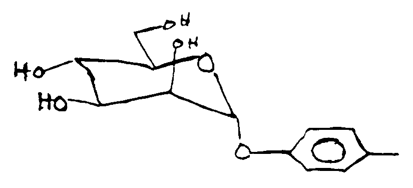
R³ is H or CH₃;
x is an integer from 0 to about 20; and
m is such that the molecular weight of the copolymer is from about 5 to about 2000 kDa.

An especially preferred mannose derivate for use in such a copolymerization has the formula: 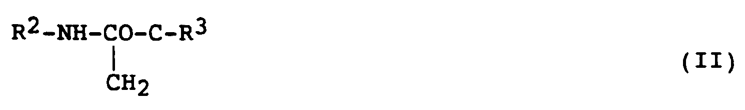
in which R² and R³ have the above mentioned meanings. For further details concerning the technique concerning such a copolymerization the published Swedish patent application 463314 is referred to, the disclosure of which is hereby incorporated by reference.

As earlier suggested, the cleansing product according to the invention can exist in form of a napkin or a product roll such as toilet paper but it can also consist of a what is called wet wipe which then suitably is enclosed in a sealed envelope.

The present invention will be illustrated further below by concrete examples which, however, are not to be interpreted as limiting the scope of the invention.

### EXAMPLE 1.

### a) Coupling of mannoside to fractogel.

Aldehydo Fractogel TSK HV 65(F) (100 g) in a moist, filtered state (0.1 M KH₂PO₄, adjusted with NaOH or HCl to pH 7), 10 µmol p-aminophenyl-α-D-mannopyranoside (A 1394, Sigma), 630 mg NaCNBH₃ (10 µmol) and 250 mg NaBH₄ are used as starting materials for the coupling reaction.

The fractogel is thoroughly dewatered and placed in a suction flask and 0.1 M phosphate buffer, pH 7 (400 ml), the mannopyranoside and the sodium cyanoborohydride are added. The flask is placed on a shaking table and a suspension is achieved by starting the shaking table. The reaction is allowed to proceed for about 4 days at room temperature.

Excess formyl groups are then destructed by the careful addition of sodium borohydride and the contents of the flask are then shaken for another hour. The gel is filtered off and thorougly washed with water. Then the gel is shaken in clean water for another hour before it is finally filtered off. The prepared gel is stored in a 25 % ethanol solution until the experiment of binding of E. coli to it.

### b) Binding of E. coli to mannose substituted fractogel.

Fifty µl of E. coli KSKP 373, 382 and 395, respectively, labelled with radioactive iodine was added to 1.5 ml phosphate buffered saline (PBS). To 0.2 ml of each bacterial suspension 0.2 ml of substituted and unsubstituted fractogel, respectively, was added. The suspensions are allowed to be incubated for 30 min on a shaking table and are then centrifuged for 10 min at 500 rpm. The supernatant is decanted off and the radioactivity in the fractogel is measured before washing, after washing with PBS and after two washings with PBS. In the present experiment the E. coli strains KSKP 373 and 382 are type 1-fimbriated while KSKP 395 lacks the type 1-fimbriae.

### RESULTS:

The results of the experiments are presented in Table I below.

**TABLE I**

| | E. coli strain | Before washing | After first washing | After second washing |
|---|---|---|---|---|
| Control gel | KSKP 373 | 1841469 | 193567 | 86162 |
| | | 18884026 | 206900 | 84472 |
| | | 1682612 | 199781 | 100471 |
| Substituted gel | KSKP 373 | 1900011 | 434393 | 272106 |
| | | 2030421 | 428443 | 264717 |
| | | 2265375 | 460703 | 239423 |
| Control gel | KSKP 382 | 1373657 | 189580 | 115216 |
| | | 1524220 | 194324 | 103543 |
| | | 1508750 | 233895 | 141118 |
| Substituted gel | KSKP 382 | 2478434 | 956399 | 624386 |
| | | 2110952 | 897985 | 677083 |
| | | 1973859 | 773461 | 541835 |
| Control gel | KSKP 395 | 1659411 | 252034 | 86400 |
| | | 1654242 | 222732 | 107824 |
| | | 1549139 | 164238 | 68923 |
| Substituted gel | KSKP 395 | 2133908 | 301827 | 164542 |
| | | 2287366 | 382092 | 183858 |
| | | 2677496 | 450601 | 187693 |

As is evident from the table, mannose substitution results in a considerably improved adherence of the bacteria compared with unsubstituted gel. Furhtermore, it is evident from the table that bacteria with type 1-fimbriae present a somewhat better adherence to the substituted gel than bacteria lacking such fimbriae.

It might be added that the structure of the mannose substituted fractogel schematically can be illustrated in the following way:

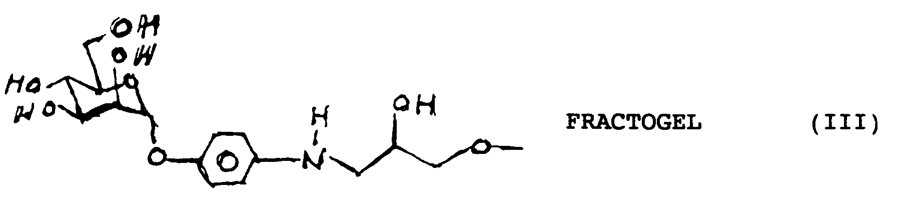

### EXAMPLE 2.

### a) Preparation of a conjugate of p-aminophenyl-α-D-mannopyranoside and human serum albumin (HSA).

p-Aminophenyl-α-D-mannopyranoside (0.1 µmol, Sigma Chemicals) is dissolved in 0.1 M phosphate buffer, pH 7.0 (50 ml), in a 50 ml conical flask and then ethanol (10 ml) is added. Then thiofosgen (0.040 ml) is added with magnetic stirring and after 10 min the magnetic bar is removed and a thorough washing is performed with distilled water (about 5 ml in total). After that, diethyl ether (about 10 ml) is added to the flask and the flask is then carefully shaken vigorously with the stopper inserted. The phases are allowed to separate and the lower water phase is then transferred to a 100 ml conical flask with a pasteur pipette. Distilled water (about 2 ml) is added to the remaining ether phase; shaking is then performed carefully and, after separaration, the new water phase is transferred to the conical flask. The conical flask is mounted on a roll evaporator and the the contents are reduced to about 2 ml.

The solution obtained is added to a protein solution prepared in advance containing HSA (162.5 mg) dissolved in borate buffer (pH 9.2, 26.3 ml), the dissolution being completed after about one hour. The pH is then adjusted to 9.5 with a 2 M solution of NaOH. The mixture is allowed to stand overnight with slow stirring and the pH is controlled to lie within the interval 8.5 to 9.5. The reaction progress is controlled by TLC. The reaction mixture is transferred to an ultrafilter which is filled with 10 to 150 ml distilled water. Ultrafiltration is performed until only some ten millilitres remain, refilling and then filtration is performed, after that another refill and filtration down to a couple of millilitres. The mixture is filtered through glass wool into a preweighed jar, whereupon lyophilization is performed.

The contents of the conjugate is either determined by sugar analysis or colorimetrically.

### b) Binding of E. coli to a paper towel coated with HSA-conjugate.

The same E. coli strains as in Example 1 were used in these experiments. Substituted and unsubstituted HSA, respectively, is dissolved in PBS (1 mg/ml). 10 µl of each solution is applied on a piece of paper punched out from a paper towel of standard quality with a perforator. When the paper has become dry 100 µl suspension of bacteria is added to the piece of paper in a test tube and incubation is performed for 30 min at 37 °C. The radioactivity is then recorded in a gamma counter and the piece of paper is washed twice with PBS. The recording in such a gamma counter is performed before the washing and after each washing. The results are shown in Table II below.

**TABLE II**

| | E. coli strain | Before washing | After first washing | After second washing |
|---|---|---|---|---|
| HSA-paper (control) | KSKP 373 | 36937 | 12773 | 5645 |
| | | 33563 | 12945 | 5982 |
| Substituted HSA-paper | KSKP 373 | 46562 | 14654 | 8288 |
| | | 42882 | 18616 | 9432 |
| HSA-paper (control) | KSKP 382 | 19113 | 13211 | 9341 |
| | | 19563 | 14647 | 10842 |
| Substituted HSA-paper | KSKP 382 | 48301 | 24067 | 15896 |
| | | 33093 | 22485 | 17597 |
| HSA-paper (control) | KSKP 395 | 16082 | 12102 | 4337 |
| | | 16164 | 7915 | 4579 |
| Substituted HSA-paper | KSKP 395 | 13819 | 7447 | 4400 |
| | | 14207 | 8709 | 4640 |

These experimental results also confirm that mannoside substitution entails a considerable improved adherence to the paper. It might be added that the conjugate in question between mannoside and HSA can be illustrated in the following way: 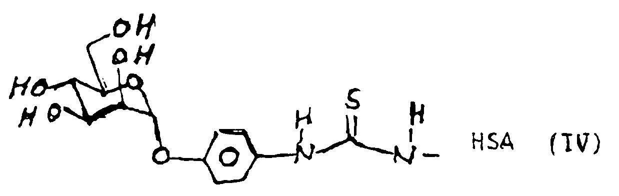
The invention is not limited to the embodiments described and shown in detail since different modifications can be made without departing from the scope of the invention. The invention is intended to be used in all such cases where a cleansing product of the kind mentioned above can be utilized for removing other bacteria than type-1 fibriated E. coli, which specifically bind to other carbohydrate derivates than those which only contain the monosaccharide mannose. The reason for not showing the corresponding embodiments here is that these other carbyhydrate derivates at present are very expensive to produce but in the future might be prepared considerably cheaper.

## Claims

1. Cleansing product for wiping for example the hands, in connection with personal hygiene, or the like, comprising a matrix of web material, **characterized** in that a carbohydrate derivate interacting with the bacteria typ-1 fimbriated E. coli is bound to the matrix for removal of said bacteria from the cleaning site by the bacteria adhering to the cleansing product.

2. Cleansing product as in claim 1, **characterized** in that said carbohydrate derivate is a mannose derivate.

3. Cleansing product as in claim 2, **characterized** in that said mannose derivate is a α-D-mannopyranoside.

4. Cleansing product as in claim 3, **characterized** in that said α-D-mannopyranoside is p-aminophenyl-α-D-mannopyranoside.

5. Cleansing product as in claim 4, **characterized** in that said α-D-mannopyranoside is bound to the matrix via its p-amino group.

6. Cleansing product as in claim 4 or 5, **characterized** in that said α-D-mannopyranoside is covalently bound to the matrix.

7. Cleansing product as in any of the preceding claims, **characterized** in that the matrix consits of cellulose based paper.

8. Cleansing product as any of claims 1 to 6, **characterized** in that the matrix consists of a composite material comprising cellulose and/or regenerated cellulose and a synthetic polymer or copolymer.

9. Cleansing product as in claim 8, **characterized** in that the matrix comprises a copolymer of a mannose derivate and an amide.

10. Cleansing product as in claim 9, **characterized** in that the copolymer has the formula: 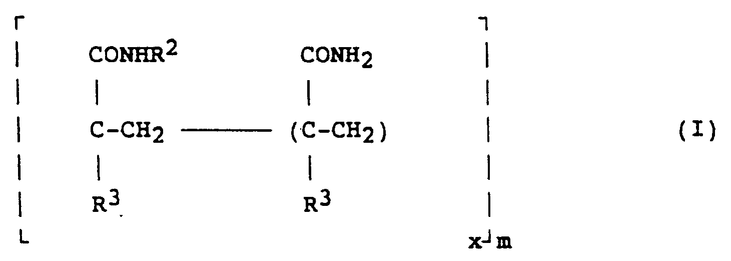 in which R² has the formula: 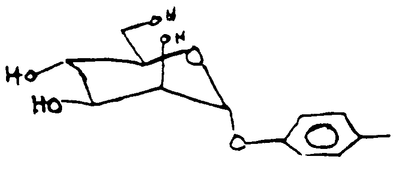 R³ is H or CH₃;
x is an integer from 0 to about 20; and
m is such that the molecular weight of the copolymer is from about 5 to about 2000 kDa.

11. Cleansing product as in any of the preceding claims, **characterized** in that the web comprises a napkin or a product roll.

12. Cleansing product as in any of the claims 1 to 10, **characterized** in that the web comprises a wet napkin enclosed in a sealed envelope.

## Patentansprüche

1. Reinigungsprodukt, z.B. zum Abwischen der Hände, im Zusammenhang mit Körperhygiene o.ä., das eine Matrix eines Gewebematerials umfaßt, dadurch **gekennzeichnet,** daß mit dieser Matrix ein Kohlenwasserstoffderivat verbunden ist, das mit Bakterien E. coli, Typ-1 befimbert, in Wechselwirkung tritt, um diese Bakterien durch das Haften der Bakterien am Reinigungsprodukt von der Reinigungsstelle zu entfernen.

2. Reinigungsprodukt nach Anspruch 1, dadurch **gekennzeichnet,** daß das Kohlenwasserstoffderivat ein Mannosederivat ist.

3. Reinigungsprodukt nach Anspruch 2, dadurch **gekennzeichnet,** daß das Mannosederivat α-D-Mannopyranosid ist.

4. Reinigungsprodukt nach Anspruch 3, dadurch **gekennzeichnet,** daß das α-D-Mannopyranosid p-Aminophenyl-α-D-mannopyranosid ist.

5. Reinigungsprodukt nach Anspruch 4, dadurch **gekennzeichnet,** daß das α-D-Mannopyranosid durch seine p-Aminogruppe an die Matrix gebunden ist.

6. Reinigungsprodukt nach Anspruch 4 oder 5, dadurch **gekennzeichnet,** daß das α-D-Mannopyranosid kovalent an die Matrix gebunden ist.

7. Reinigungsprodukt nach einem der vorstehenden Ansprüche, dadurch **gekennzeichnet,** daß die Matrix aus Papier auf Cellulosebasis besteht.

8. Reinigungsprodukt nach einem der Ansprüche 1 bis 6, dadurch **gekennzeichnet,** daß die Matrix aus einem Verbundmaterial besteht, das Cellulose und/oder regenerierte Cellulose und ein synthetisches Polymer oder Copolymer umfaßt.

9. Reinigungsprodukt nach Anspruch 8, dadurch **gekennzeichnet,** daß die Matrix ein Copolymer eines Mannosederivats und eines Amids umfaßt.

10. Reinigungsprodukt nach Anspruch 9, dadurch **gekennzeichnet,** daß das Copolymer die Formel aufweist: 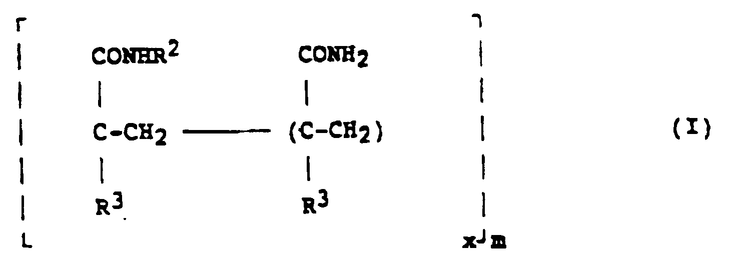 worin R² die Formel hat: 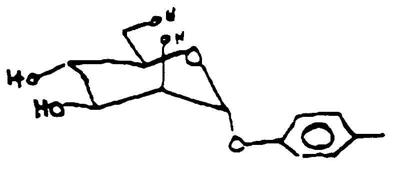 R³ H oder CH₃ ist;
x eine ganze Zahl von 0 bis etwa 20 ist; und
m derart ist, daß das Molekulargewicht des Copolymers etwa 5 bis etwa 2000 kDa beträgt.

11. Reinigungsprodukt nach einem der vorstehenden Ansprüche, dadurch **gekennzeichnet,** daß das Gewebe eine Serviette oder ein Rollenprodukt umfaßt.

12. Reinigungsprodukt nach einem der Ansprüche 1 bis 10, dadurch **gekennzeichnet,** daß das Gewebe eine feuchte Serviette umfaßt, die in einem verschlossenen Umschlag enthalten ist.

## Revendications

1. Produit assainissant pour l'essuyage des mains, par exemple, en rapport avec l'hygiène personnelle, ou autre, comportant une matrice à base de matériau tissé, caractérisé en ce qu'un dérivé de type carbohydrate interagissant avec la bactérie E. coli type-1 fimbriée est lié à la matrice pour éliminer ladite bactérie de l'endroit à nettoyer par adhésion de la bactérie sur le produit assainissant.

2. Produit assainissant selon la revendication 1, caractérisé en ce que ledit dérivé de type carbohydrate est un dérivé du mannose.

3. Produit assainissant selon la revendication 2, caractérisé en ce que ledit dérivé du mannose est un α-D-mannopyranoside.

4. Produit assainissant selon la revendication 3, caractérisé en ce que ledit α-D-mannopyranoside est le p-aminophényl-α-D-mannopyranoside.

5. Produit assainissant selon la revendication 4, caractérisé en ce que ledit α-D-mannopyranoside est lié à la matrice via son groupe p-amino.

6. Produit assainissant selon la revendication 4 ou 5, caractérisé en ce que ledit α-D-mannopyranoside est lié à la matrice par liaison de covalence.

7. Produit assainissant selon l'une quelconque des revendications précédentes, caractérisé en ce que la matrice consiste en un papier à base de cellulose.

8. Produit assainissant selon l'une quelconque des revendications de 1 à 6, caractérisé en ce que la matrice consiste en un matériau composite comportant de la cellulose et/ou de la cellulose régénérée et un polymère ou copolymère synthétique.

9. Produit assainissant selon la revendication 8, caractérisé en ce que la matrice comporte un copolymère d'un dérivé du mannose et d'un amide.

10. Produit assainissant selon la revendication 9, caractérisé en ce que le copolymère a la formule: 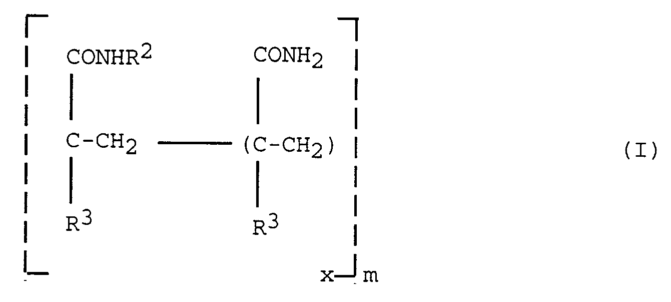 dans laquelle R² a la formule: 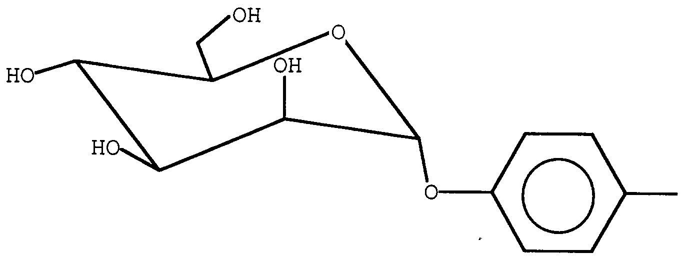 R³ est H ou CH₃,
x est un nombre entier de 0 à environ 20 et
m est tel que le poids moléculaire du copolymère soit compris entre environ 5 et environ 2000 kDa.

11. Produit assainissant selon l'une quelconque des revendications précédentes, caractérisé en ce que le tissu comprend une serviette ou un rouleau.

12. Produit assainissant selon l'une quelconque des revendications de 1 à 10, caractérisé en ce que le tissu comprend une serviette tissée inclue dans une enveloppe scellée.
